(19) **Europäisches Patentamt**
**European Patent Office**
**Office européen des brevets**

(11) **EP 2 319 567 B1**

(12) **EUROPEAN PATENT SPECIFICATION**

(45) Date of publication and mention
of the grant of the patent:
**07.01.2015 Bulletin 2015/02**

(51) Int Cl.:
***A61M 16/01*** *(2006.01)*

(21) Application number: **10189576.1**

(22) Date of filing: **01.11.2010**

(54) **Method and system for controlling a ventilator**

Verfahren und System zur Steuerung eines Ventilators

Procédé et système de contrôle d'un ventilateur

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.11.2009 US 613726**

(43) Date of publication of application:
**11.05.2011 Bulletin 2011/19**

(73) Proprietor: **General Electric Company**
**Schenectady, NY 12345 (US)**

(72) Inventor: **Viertio-Oja, Hanna Elina**
**00510, Helsinki (FI)**

(74) Representative: **Illingworth-Law, William Illingworth**
**GPO Europe**
**GE International Inc.**
**The Ark**
**201 Talgarth Road**
**Hammersmith**
**London W6 8BJ (GB)**

(56) References cited:
**WO-A1-2006/005433      WO-A1-2006/131149**
**WO-A1-2010/150264      US-A1- 2008 236 582**

EP 2 319 567 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

This disclosure relates generally to a system for controlling a level of ventilatory support from a ventilator for the purpose of weaning a patient from the ventilator.

BACKGROUND OF THE INVENTION

WO 2006/005433 and WO 2006/131149 disclose ventilators generating breathing gas flows in response to breathing pattern changes.

Weaning a patient from artificial ventilation is among the most difficult questions regarding intensive care ventilation. Patients encountering weaning problems have often been ventilated for more than 48 hours before the ventilator weaning can be considered. While on the ventilator, some patients develop ventilator dependency and they cannot maintain breathing on their own. This dependency may prolong the patient's stay on the ventilator. Prolonged ventilation increases the risk of developing lung inflammation known as ventilator induced lung injury (VILI). Even worse, inflammation may spread from the lungs to other organs resulting in multiple-organ failure. Mortality of patients experiencing multiple-organ failure is high. Therefore, minimizing the patient's stay on the ventilator is one of the primary goals for an intensive care unit.

It has been experimentally shown that daily spontaneous breathing trials by the patient significantly reduce the number of days that a patient spends in mechanical ventilation. Until recently, weaning from ventilation and a patient's sedation level have been considered separately. However, research has shown that it may be beneficial to consider the patient's sedation level in order to optimize the timing of the spontaneous breathing trials. Specifically, it would be preferable to perform the spontaneous breathing trials when the patient is at a relatively alert sedation level.

Using conventional technology, the combination of using the patient's sedation level to determine the best times for performing spontaneous breathing trials would require almost continuous attendance of a caregiver during each spontaneous breathing trial. Due to staff and budget constraints, it would be challenging for the intensive care unit to perform this protocol for all the patients on artificial ventilation.

Thus, there is a need for an automated technique utilizing the patient's sedation level to determine the best times for spontaneous breathing trials without requiring significant involvement of the intensive care unit staff.

BRIEF DESCRIPTION OF THE INVENTION

The above-mentioned shortcomings, disadvantages and problems are addressed herein which will be understood by reading and understanding the following specification.

The present invention provides an intensive care system as defined in claim 1.

Various other features, objects, and advantages of the invention will be made apparent to those skilled in the art from the accompanying drawings and detailed description thereof.

BRIEF DESCRIPTION OF THE DRAWINGS

FIGURE 1 is a schematic diagram illustrating an intensive care system in accordance with an embodiment;

FIGURE 2 is a flow chart in accordance with an embodiment; and

FIGURE 3 is a graph of responsiveness versus time in accordance with an embodiment.

DETAILED DESCRIPTION OF THE INVENTION

In the following detailed description, reference is made to the accompanying drawings that form a part hereof, and in which is shown by way of illustration specific embodiments that may be practiced. These embodiments are described in sufficient detail to enable those skilled in the art to practice the embodiments, and it is to be understood that other embodiments may be utilized and that logical, mechanical, electrical and other changes may be made without departing from the scope of the embodiments. The following detailed description is, therefore, not to be taken as limiting the scope of the invention.

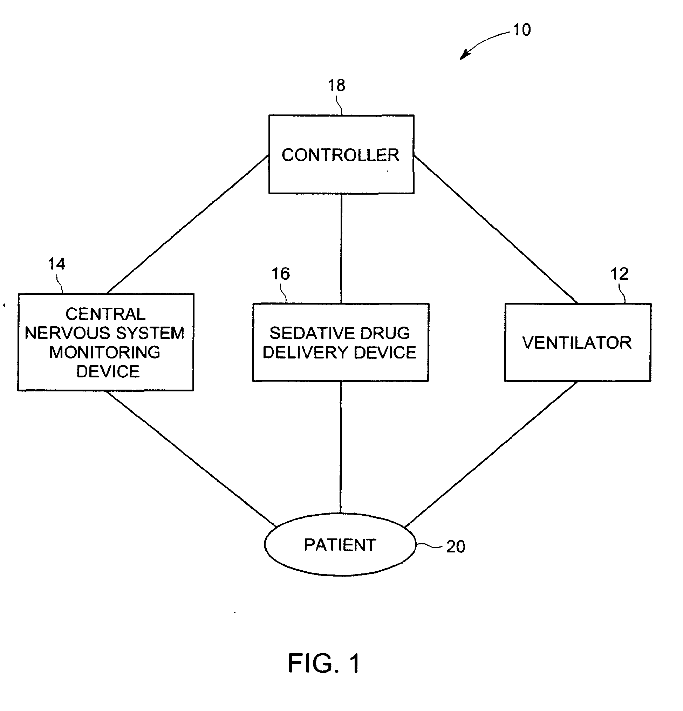

Figure 1 is a schematic diagram illustrating an intensive care system 10 in accordance with an embodiment. The intensive care system 10 includes a ventilator 12, a central nervous system monitoring device 14, a sedative drug delivery device 16, and a controller 18. The embodiment shown in Figure 1 includes a patient 20 attached to the central nervous system monitoring device 14, the sedative drug delivery device 16 and the ventilator 12. The ventilator 12 is designed to mechanically move air into the patient's lungs. The ventilator 12 may be of a conventional design. According to an embodiment, the sedative drug delivery device 16 may comprise a pump designed to administer a sedative drug to the patient 20 intravenously. According to another embodiment, the sedative drug delivery device 16 may comprise a gas delivery system that is designed to control an amount of a sedative gas delivered to the patient 20.

The central nervous system monitoring device 14 comprises a device configured to monitor a central nervous system parameter of the patient 20. According to an embodiment, the central nervous system monitoring device 14 may comprise an electroencephalograph, which will be referred to as an EEG hereinafter, or an electromyograph, which will be referred to as an EMG hereinafter. The EEG records electrical activity of the patient's brain as measured at the patient's scalp. It is possible to determine a sedation level of the patient 20 by analyzing one or more EEG parameters collected with an EEG. The EEG parameters are examples of central nervous system parameters in accordance with an embodiment. As a general rule, a high sedation level tends to be correlated with a low level of electrical activity and a low sedation level tends to be correlated with a high level electrical activity as measured with an EEG. In other words, the patient 20 would typically have a high level of electrical activity during an alert period. For purposes of this disclosure, the term "alert period" is defined to include a period of time where the patient is at a relatively low sedation level. In other words, an alert period corresponds to a period of time where the patient is relatively responsive. The standard for defining an alert period may vary on a patient-to-patient basis. One of the keys in identifying an alert period is to monitor a central nervous system parameter, such as an EEG parameter, over a period of time to determine periods of relative alertness. For example, according to an embodiment, the central nervous system parameter may be monitored for a period of 24 hours. After monitoring for 24 hours, the periods of relative alertness may be identified. The thresholds for determining an alert period may thus be based on the results of monitoring the patient over an extended period of time.

An EMG, meanwhile, is used to test the electrical activity of the patient's muscles. One or more EMG parameters collected with the EMG may also be analyzed to determine the sedation level of the patient 20. The EMG parameters are also examples of central nervous system parameters in accordance with an embodiment. It is possible to determine a sedation level of the patient 20 by analyzing one or more EMG parameters collected with an EMG. According to another embodiment, the central nervous system monitoring device may acquire data from an EEG or an EMG and then calculate a central nervous system parameter called responsiveness. The responsiveness parameter may also be used to determine a sedation level. The responsiveness parameter will be discussed in more detail hereinafter.

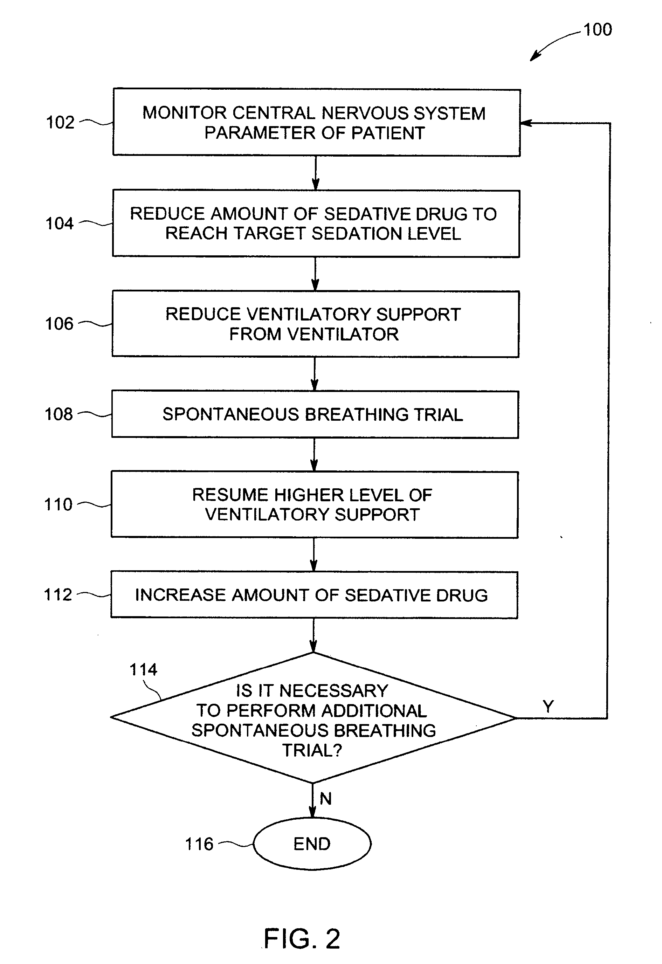

Figure 2 is a flow chart showing the method 100 in accordance with an embodiment. The individual blocks 102-116 represent steps that may be performed in accordance with the method 100. Additional embodiments may perform the steps shown in a different sequence and/or additional embodiments may include additional steps not shown in Figure 2. Other embodiment may not perform all the steps shown in Figure 2. The technical effect of the method 100 is the automatic controlling of a ventilator in order to allow the patient 20 (shown in Figure 1) to perform a spontaneous breathing trial at a relatively low sedation level.

Referring to Figure 1 and Figure 2, at step 102, the central nervous system monitoring device 14 begins to monitor a central nervous system parameter. The central nervous system monitoring device 14 may continuously acquire data from the patient, or the central nervous system monitoring device 14 may only start acquiring data after a clinician provides instructions through a user interface to begin a weaning trial. For example, the clinician may select a menu item called "weaning trial" from a list of menu options. According to an exemplary embodiment, the central nervous system parameter may be a patient's responsiveness. The central nervous system monitoring device 14 may comprise an EEG and the controller 18 may use the EEG to determine the patient's responsiveness. For the purposes of this disclosure, responsiveness R(t) may be determined according to equation (1) as follows:

$$Rp(t) = \frac{1}{T2} \int_{t-T2}^{t} \log(\frac{\Delta P(u)}{P_0}) du, \quad (1),$$

where

$$\Delta P(u) = P_{EEG/EMG}(u) - \min_{(u-T1) \le v \le u} P_{EEG/EMG}(v)$$

where $P_{EEG/EMG}(ti)$ refers to high-frequency EEG or EMG power in a short time window ti computed over the desired frequency range, such as a range of 50 Hz to 150 Hz and $P_0$ is a fixed reference value for the power change. In the above example, the length of this time window corresponds to one epoch (5 seconds according to an embodiment), while T2 may be 30 minutes, and T1 may be 1 minute. However, the values of the desired frequency range, the length of the time window, the value of T1 and the value of T2 may all be different according to additional embodiments. It should be appreciated by those skilled in the art that additional techniques may also be used to determine the sedation level of the patient 20.

One advantage of monitoring a central nervous system parameter such as responsiveness is that the controller 18 can ensure that the spontaneous breathing trials do not occur during periods of time when the patient is sleeping. Statistically, the patient is likely to experience a faster recovery if they are allowed to get sufficient rest. An automated system that respects the patient's sleep cycles is likely to promote a faster recovery.

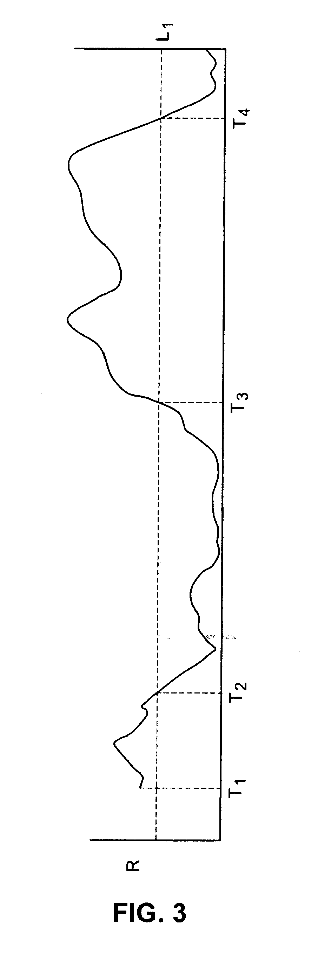

Figure 3 is a chart showing how the responsiveness ( R ) of a patient varies over a number of hours. The responsiveness is determined using equation 1 as was described hereinabove. The responsiveness correlating to the level indicated by L1 represents a target level of responsiveness for the patient. The target level of responsiveness corresponds to a target sedation level for the patient. During the time period between T1 and T2, the patient is at a responsiveness level above the threshold. The patient is also at a responsiveness level above the threshold during the time period between T3 and T4. However, the chart clearly shows that the patient is at a significantly lower responsiveness during the time period from T2 to T3. Based on the information represented in Figure 3, it would be best to perform one or more spontaneous breathing trials during the time period between T1 and T2 or during the time period between T3 and T4. Additionally, the information represented in the chart may also be used to establish a baseline value of responsiveness for a particular patient. The baseline may be used by the controller 18 (shown in Figure 1) to help determine the amount of sedative drug to be administered to the patient as will be discussed in detail hereinafter.

Referring to Figure 1 and Figure 2, at step 104, the controller 18 instructs the sedative drug delivery device 16 to reduce an amount of sedative drug administered to the patient 20. The central nervous system monitoring device 14 continues to collect data that can be used to determine the sedation level of the patient 20 during step 104. The controller 18 is able to determine the proper amount of sedative drug administered to the patient 20 based on feedback received from the central nervous system monitoring device 14. By monitoring the sedation level through a central nervous system parameter such as responsiveness and adjusting the amount of sedative drug administered to the patient 20, it may be possible to adjust the patient's sedation level to one that enables optimally effective spontaneous breathing trials. The target sedation level may be determined based on empirical data or it may be patient-specific. For example, by monitoring a patient's change in sedation level over one or more days, the controller 18 may be able to calculate a target sedation level. Based on studies so far, it appears that a responsiveness of about 50 as measured on a linear scale ranging from 0 to 100 is close to optimal for most intensive care unit patients.

According to another embodiment, the method 100 may skip step 104. Instead of controlling a sedative drug delivery device, the controller 18 would instead rely on data from the central nervous system monitoring device 14 in order to identify when the patient 20 is in an alert period. For example, by monitoring the sedation level of the patient 20 with the central nervous system monitoring device 14 for a period of time, such as a number of hours, the controller 18 may be able to identify an alert period. By monitoring the sedation level over a period of time, the controller 18 may be able to identify when the patient 20 is at a sedation level that is low relative to the previous number of hours and/or days. It should be understood that the threshold defining the target sedation level may change during the course of treatment for the patient 20.

After the target sedation level has been reached, the controller 18 instructs the ventilator 12 to reduce the level of ventilatory support to the patient at step 106. The level of ventilatory support may be reduced according to any known protocol. For example, according to an exemplary embodiment, reducing the level of ventilatory support may comprise decreasing a level of pressure support provided by the ventilator 12. The pressure support may be decreased in either a step-wise or a generally continuous manner. During step 106, the controller 18 monitors the patient 20 for the purpose of detecting if the patient 20 has an adverse reaction to the lower level of ventilatory support. One technique for monitoring the patient 20 for an adverse reaction will be discussed hereinafter. Decreasing the level of ventilatory support may include the control of other parameters such as reducing a duration of pressure support during the patient's inspiration. Reducing the level of ventilatory support may comprise other techniques in accordance with additional embodiments.

Still referring to Figure 1 and Figure 2, at step 108, the patient 20 performs a spontaneous breathing trial at the lower level of ventilatory support. Depending upon the needs of the individual patient, the spontaneous breathing trials may be performed without any ventilatory support. As the patient 20 performs the spontaneous breathing trial, a gas-analysis monitor (not shown) attached to the ventilator may measure a gas exchange parameter such as a ratio of $VCO_2$ to $VO_2$. A healthy patient capable of maintaining normal lung function is able to maintain a nearly constant ratio of $VCO_2$ to $VO_2$ as the ventilatory support is reduced. However, a patient who is not able to maintain normal ventilation will exhibit a change in the $VCO_2$ to $VO_2$ ratio as the ventilatory support is reduced. This is because $O_2$ ($VO_2$) consumption for a patient not capable of maintaining normal ventilation increases more rapidly than $CO_2$-production ($VCO_2$) when the patient tries to maintain his/her breathing at a certain level of ventilatory support. If the patient's $VCO_2$ to $VO_2$ ratio falls below a critical threshold, the controller 18 may abort the spontaneous breathing trial and instruct the ventilator 12 to increase the ventilatory support provided to the patient 20. If the $VCO_2$ to $VO_2$ ratio falls below a dangerous level, the controller 18 may optionally activate an alarm and alert a caregiver. According to additional embodiments, the controller 18 may also acquire signals from additional patient monitoring devices in order to detect if the patient is experiencing an adverse consequence due to the reduced sedation. For example, the controller 18 may receive signals from patient monitoring devices such as an electrocardiograph, a movement sensor, a pulse oximeter, or a spirometer. If the controller 18 detects an adverse reaction from any of the patient monitoring devices, the controller 18 may instruct the ventilator 12 to increase the level of ventilatory support to the patient 20. The controller 18 may also instruct the sedative drug delivery device 16 to increase the amount of sedative drug administered to the patient.

Still referring to step 108, the spontaneous breathing trial typically lasts from 1 minute to 10 minutes in length. However, embodiments of the invention may use spontaneous breathing trials that are either shorter than 1 minute or longer than 10 minutes. It should be appreciated by those skilled in the art that a relatively healthy patient may have a longer breathing trial than a patient who is less healthy. The duration of the spontaneous breathing trial may be determined automatically based on the patient's ability to maintain the proper VO2 to VCO2 ratio at a specific level of ventilatory support or the duration of the spontaneous breathing trial may be manually set by a caregiver.

During the spontaneous breathing trial at step 108, the central nervous system monitoring device 14 may continue to monitor the sedation level of the patient 20. The controller 18 may communicate with the sedative drug delivery device 16 to keep the patient 20 at a target sedation level during the spontaneous breathing trial. However, if the sedation level of the patient 20 falls outside of the desired range even with the control of the sedative drug delivery device 16, the spontaneous breathing trial may be aborted. Thus far, experimental data have shown that spontaneous breathing trials are most effective when the patient 20 is at a sedation level consistent with a period of relative alertness. If the controller 18 and the sedative drug delivery device 16 cannot maintain the patient 20 within the proper range of sedation levels, it is generally better to resume a higher level of ventilatory support.

Still referring to Figures 1 and 2, at step 110, the controller 18 instructs the ventilator 12 to resume a higher level of ventilatory support. According to an embodiment, the higher level of ventilatory support may comprise an increased level of pressure support from the ventilator 12. The ventilator 12 may increase the level of pressure support to that which was used before the spontaneous breathing trial, or the ventilator 12 may increase ventilator support to a different level of pressure support. For example, the controller 18 may instruct the ventilator 12 to increase to a level of pressure support that is lower than the level of pressure support that was used before as part of a weaning process.

At step 112, the controller 18 instructs the sedative drug delivery device 16 to increase an amount of the sedative drug administered to the patient 20. The central nervous system monitoring device 14 may monitor a central nervous system parameter such as responsiveness while the sedative drug delivery device 16 increases the amount of sedative drug administered to the patient 20. By monitoring the feedback from the central nervous system monitoring device 14, the controller 18 may determine the appropriate amount of sedative drug to administer to the patient 20.

In accordance with an embodiment where the controller 18 does not control a sedative drug delivery device, the method 100 may optionally skip step 112 and proceed from step 110 directly to step 114.

At step 114, the controller 18 determines if it is necessary to perform an additional spontaneous breathing trial. It may be desirable to perform one or more spontaneous breathing trials per day. In accordance with an embodiment, the controller 18 may wait approximately 24 hours before beginning the next spontaneous breathing trial. When it is time for the next spontaneous breathing trial, the method 100 returns to step 102 and steps 102-114 are repeated. If it is not necessary to perform another spontaneous breathing trial, the method 100 advances to step 116 and ends. After finishing one or more spontaneous breathing trials, the controller 18 may compile a summary regarding the patient's performance during the spontaneous breathing trial or trials. The summary may include trend information of parameters such as VO2, VCO2, respiration rate, and minute volumes. According to another embodiment, the clinician may decide if an additional spontaneous breathing trial is needed or if the patient is ready to be taken off the ventilator based on the summary.

This written description uses examples to disclose the invention, including the best mode, and also to enable any person skilled in the art to practice the invention, including making and using any devices or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal language of the claims.

Various aspects of the present disclosure are defined in the following numbered clauses:

1. A method for automatically controlling a ventilator comprising:

monitoring a central nervous system parameter; and

automatically adjusting a level of ventilatory support based on a value of the central nervous system parameter.

2. The method of clause 1, wherein said automatically adjusting the level of ventilatory support comprises adjusting a level of pressure support from the ventilator.

3. The method of clause 1 or clause 2, wherein said automatically adjusting the level of ventilatory support comprises adjusting a duration of pressure support during a patient's inspiration.

4. The method of any preceding clause, further comprising detecting an alert period based on the value of the central nervous system parameter.

5. The method of clause 4, wherein said automatically adjusting the level of ventilatory support comprises reducing the level of ventilatory support during the alert period.

6. The method of clause 5, further comprising monitoring for an adverse reaction at the reduced level of ventilatory support.

7. The method of any preceding clause, wherein said monitoring the central nervous system parameter comprises monitoring an EEG parameter or an EMG parameter.

8. The method of any preceding clause, wherein said monitoring the central nervous system parameter comprises monitoring a patient's responsiveness.

9. A method for weaning a patient from a ventilator comprising:

monitoring a central nervous system parameter for the purpose of determining a sedation level;

reducing an amount of a sedative drug administered to the patient in order to reach a target sedation level;

automatically reducing a level of ventilatory support to a lower level of ventilatory support at the target sedation level; and

conducting a spontaneous breathing trial at the lower level of ventilatory support.

10. The method of clause 9, further comprising increasing the level of ventilatory support to the patient after the spontaneous breathing trial.

11. The method of clause 9 or clause 10, further comprising automatically increasing the amount of the sedative drug administered to the patient after the spontaneous breathing trial.

12. The method of any of clauses 9 to 11, further comprising monitoring a gas exchange parameter during the spontaneous breathing trial.

13. The method of clause 12, further comprising automatically increasing the level of ventilatory support after detecting that the gas exchange parameter is outside of a desired range.

14. The method of clause 13, further comprising automatically increasing the amount of the sedative drug administered to the patient after detecting that the gas exchange parameter is outside of the desired range.

15. A intensive care system comprising:

a ventilator;

a central nervous system monitoring device; and

a controller connected to the ventilator and the central nervous system monitoring device, said controller configured to:

monitor a central nervous system parameter acquired with the central nervous system monitoring device for the purpose of determining a sedation level;

detect when the sedation level reaches a target sedation level;

reduce a level of ventilatory support to a lower level of ventilatory support when the sedation level reaches the target sedation level;

allow the patient to perform a spontaneous breathing trial at the lower level of ventilatory support; and

increase the level of ventilatory support after the spontaneous breathing trial.

16. The intensive care system of clause 15, wherein the central nervous system monitoring device comprises an electroencephalograph or an electromyograph.

17. The intensive care system of clause 15 or clause 16, further comprising a sedative drug delivery device controlled by the controller.

18. The intensive care system of clause 17, wherein the controller is further configured to lower an amount of a sedative drug administered to the patient from the sedative drug delivery device to help the patient attain the target sedation level.

19. The intensive care system of any of clauses 15 to 18, wherein the controller is further configured to receive information about an 02 level and a CO2 level during the spontaneous breathing trail.

20. The intensive care system of any of clauses 15 to 19, wherein the controller is further configured to operate automatically.

**Claims**

1.  An intensive care system (10) comprising:

    a ventilator (12);
    a central nervous system monitoring device (14); and
    a controller (18) connected to the ventilator (12) and the central nervous system monitoring device (14), said controller (18) configured to:

    monitor a central nervous system parameter acquired with the central nervous system monitoring device (14) for the purpose of determining a sedation level;
    detect when the sedation level reaches a target sedation level;
    reduce a level of ventilatory support to a lower level of ventilatory
    support when the sedation level reaches the target sedation level; and allow the patient to perform a spontaneous breathing trial at the lower level of ventilatory support.

2.  The intensive care system (10) of claim 1, wherein the controller (18) is further configured to increase the level of ventilatory support after the spontaneous breathing trial.

3.  The intensive care system (10) of claim 1 or claim 2, wherein the controller (18) is further configured to monitor a gas exchange parameter during the spontaneous breathing trial.

4.  The intensive care system (10) of claim 3, wherein the controller (18) is further configured to automatically increase the level of ventilatory support after detecting that the gas exchange parameter is outside of a desired range.

5.  The intensive care system (10) of any one of the preceding claims, further comprising a sedative drug delivery device controlled by the controller (18).

6.  The intensive care system (10) of any preceding claim, wherein the controller (18) is configured to adjust a level of pressure support from the ventilator (12).

7.  The intensive care system (10) of any preceding claim, wherein the controller (18) is configured to adjust a duration of pressure support from the ventilator (12) during a patient's inspiration.

8. The intensive care system (10) of any preceding claim, wherein the controller (18) is further configured to detect an alert period based on the value of the central nervous system parameter.

9. The intensive care system (10) of claim 8, wherein the controller (18) is further configured to reduce the level of ventilatory support during the alert period.

10. The intensive care system (10) of claim 9, wherein the controller (18) is further configured to monitor for an adverse reaction at the reduced level of ventilatory support.

11. The intensive care system (10) of any preceding claim, wherein the central nervous system monitoring device (14) comprises an electroencephalograph.

12. The intensive care system (10) of any preceding claim, wherein the central nervous system monitoring device (14) comprises an electromyograph.

13. The intensive care system (10) of any preceding claim, wherein the controller (18) is configured to generate the central nervous system parameter based on information from the central nervous system monitoring device (14).

14. The intensive care system (10) of any preceding claim, wherein the controller (18) is configured to monitor a patient's responsiveness.


**Patentansprüche**

1. Intensivmedizinsystem (10), welches Folgendes umfasst:

ein Beatmungsgerät (12);
eine Überwachungsvorrichtung für das zentrale Nervensystem (14); und
ein Steuergerät (18), angeschlossen an das Beatmungsgerät (12) und die Überwachungsvorrichtung für das zentrale Nervensystem (14), wobei das Steuergerät (18) dafür konfiguriert ist, um:

einen Parameter des zentralen Nervensystems zu überwachen,
der mit der Überwachungsvorrichtung für das zentrale Nervensystem (14) erfasst worden ist, um eine Sedierungsstufe zu bestimmen;
zu bestimmen, wann die Sedierungsstufe eine Zielsedierungsstufe erreicht;
eine Stufe der unterstützenden Beatmung auf eine niedrigere Stufe der unterstützenden Beatmung herabzusetzen, wenn die Sedierungsstufe die Zielsedierungsstufe erreicht; und

es dem Patienten zu ermöglichen, auf der niedrigeren Stufe der unterstützenden Beatmung einen Spontanatmungsversuch zu unternehmen.

2. Intensivmedizinsystem (10) nach Anspruch 1, wobei das Steuergerät (18) ferner konfiguriert ist, um die Stufe der unterstützenden Beatmung nach dem Spontanatmungsversuch zu erhöhen.

3. Intensivmedizinsystem (10) nach Anspruch 1 oder 2, wobei das Steuergerät (18) ferner konfiguriert ist, während des Spontanatmungsversuchs einen Gasaustauschparameter zu überwachen.

4. Intensivmedizinsystem (10) nach Anspruch 3, wobei das Steuergerät (18) ferner konfiguriert ist, die Stufe der unterstützenden Beatmung automatisch weiter zu erhöhen, wenn festgestellt worden ist, dass der Parameter außerhalb eines Sollbereichs liegt.

5. Intensivmedizinsystem (10) nach einem der vorhergehenden Ansprüche, ferner umfassend ein von dem Steuergerät (18) geregeltes Verabreichungssystem für Beruhigungsmittel.

6. Intensivmedizinsystem (10) nach einem der vorhergehenden Ansprüche, wobei das Steuergerät (18) konfiguriert ist, eine Stufe der Druckunterstützung durch das Beatmungsgerät (12) anzupassen.

7. Intensivmedizinsystem (10) nach einem der vorhergehenden Ansprüche, wobei das Steuergerät (18) konfiguriert ist, eine Dauer der Druckunterstützung durch das Beatmungsgerät (12) während einer Inspiration des Patienten anzupassen.

8. Intensivmedizinsystem (10) nach einem der vorhergehenden Ansprüche, wobei das Steuergerät (18) ferner konfiguriert ist, einen Alarmzeitraum auf der Grundlage des Parameters des zentralen Nervensystems zu erfassen.

9. Intensivmedizinsystem (10) nach Anspruch 8, wobei das Steuergerät (18) ferner konfiguriert ist, um die Stufe der unterstützenden Beatmung während des Alarmzeitraums zu verringern.

10. Intensivmedizinsystem (10) nach Anspruch 9, wobei das Steuergerät (18) ferner konfiguriert ist, um auf der niedrigeren Stufe der unterstützenden Beatmung negative Reaktionen zu erkennen.

11. Intensivmedizinsystem (10) nach einem der vorhergehenden Ansprüche, wobei die Überwachungsvorrichtung für das zentrale Nervensystem (14) einen Elektroenzephalographen umfasst.

12. Intensivmedizinsystem (10) nach einem der vorhergehenden Ansprüche, wobei die Überwachungsvorrichtung für das zentrale Nervensystem (14) einen Elektromyographen umfasst.

13. Intensivmedizinsystem (10) nach einem der vorhergehenden Ansprüche, wobei das Steuergerät (18) konfiguriert ist, um auf der Datengrundlage der Überwachungsvorrichtung für das zentrale Nervensystem (14) den Parameter des zentralen Nervensystems zu erstellen.

14. Intensivmedizinsystem (10) nach einem der vorhergehenden Ansprüche, wobei das Steuergerät (18) konfiguriert ist, die Ansprechbarkeit eines Patienten zu überwachen.


## Revendications

1. Un système de soins intensifs (10) comprenant :

   un ventilateur (12) ;
   un appareillage de surveillance du système nerveux central (14) ; et
   un contrôleur (18) connecté au ventilateur (12) et à l'appareillage de surveillance du système nerveux central (14), ledit contrôleur (18) étant agencé pour :

      surveiller un paramètre du système nerveux central acquis avec l'appareillage de surveillance du système nerveux central (14) dans le but de déterminer un niveau de sédation ;
      détecter quand le niveau de sédation atteint un niveau de sédation cible ;
      réduire un niveau de support ventilatoire à un niveau de support ventilatoire inférieur quand le niveau de sédation atteint le niveau de sédation cible ; et

   permettre au patient de réaliser un test de respiration spontanée à un niveau de support ventilatoire inférieur.

2. Le système de soins intensifs (10) de la revendication 1, dans lequel le contrôleur (18) est agencé en outre pour augmenter le niveau de support ventilatoire après le test de respiration spontanée.

3. Le système de soins intensifs (10) de la revendication 1 ou de la revendication 2, dans lequel le contrôleur (18) est agencé en outre pour surveiller le paramètre d'échange de gaz au cours du test de respiration spontanée.

4. Le système de soins intensifs (10) de la revendication 3, dans lequel le contrôleur (18) est agencé en outre pour augmenter automatiquement le niveau de support ventilatoire après détection que le paramètre d'échange de gaz est en-dehors d'une plage désirée.

5. Le système de soins intensifs (10) d'une quelconque des revendications précédentes comprenant en outre un dispositif de distribution de médicaments sédatifs sous le contrôle du contrôleur (18).

6. Le système de soins intensifs (10) d'une quelconque revendication précédente, dans lequel le contrôleur (18) est agencé pour ajuster un niveau de support de pression à partir du ventilateur (12).

7. Le système de soins intensifs (10) d'une quelconque revendication précédente, dans lequel le contrôleur (18) est agencé pour ajuster la durée de support de pression à partir du ventilateur (12) pendant l'inspiration du patient.

8. Le système de soins intensifs (10) d'une quelconque revendication précédente, dans lequel le contrôleur (18) est agencé en outre pour détecter une période d'alerte basée sur la valeur du paramètre du système nerveux central.

9. Le système de soins intensifs (10) de la revendication 8, dans lequel le contrôleur (18) est agencé en outre pour réduire le niveau de support ventilatoire pendant la période d'alerte.

10. Le système de soins intensifs (10) de la revendication 9, dans lequel le contrôleur (18) est agencé en outre à la surveillance d'une réaction défavorable au niveau réduit de support ventilatoire.

11. Le système de soins intensifs (10) d'une quelconque revendication précédente, dans lequel le dispositif de surveillance du système nerveux central (14) comprend un électroencéphalographe.

12. Le système de soins intensifs (10) d'une quelconque revendication précédente, dans lequel le dispositif de surveillance du système nerveux central (14) comprend un électromyographe.

13. Le système de soins intensifs (10) d'une quelconque revendication précédente, dans lequel le contrôleur (18) est agencé pour générer le paramètre du système nerveux central basé sur les informations du dispositif de surveillance du système nerveux central (14).

14. Le système de soins intensifs (10) d'une quelconque revendication précédente, dans lequel le contrôleur (18) est agencé pour surveiller la sensibilité d'un patient.

FIG. 1

100

```
102 ── ┌─────────────────────────────────────┐
        │   MONITOR CENTRAL NERVOUS SYSTEM    │◄──
        │      PARAMETER OF PATIENT           │   │
        └─────────────────────────────────────┘   │
                        │                          │
                        ▼                          │
104 ── ┌─────────────────────────────────────┐   │
        │  REDUCE AMOUNT OF SEDATIVE DRUG TO  │   │
        │    REACH TARGET SEDATION LEVEL      │   │
        └─────────────────────────────────────┘   │
                        │                          │
                        ▼                          │
106 ── ┌─────────────────────────────────────┐   │
        │      REDUCE VENTILATORY SUPPORT     │   │
        │          FROM VENTILATOR            │   │
        └─────────────────────────────────────┘   │
                        │                          │
                        ▼                          │
108 ── ┌─────────────────────────────────────┐   │
        │      SPONTANEOUS BREATHING TRIAL    │   │
        └─────────────────────────────────────┘   │
                        │                          │
                        ▼                          │
110 ── ┌─────────────────────────────────────┐   │
        │         RESUME HIGHER LEVEL OF      │   │
        │          VENTILATORY SUPPORT        │   │
        └─────────────────────────────────────┘   │
                        │                          │
                        ▼                          │
112 ── ┌─────────────────────────────────────┐   │
        │    INCREASE AMOUNT OF SEDATIVE DRUG │   │
        └─────────────────────────────────────┘   │
                        │                          │
                        ▼                          │
        114                                        │
             ◇ IS IT NECESSARY ◇                  │
             TO PERFORM ADDITIONAL          Y      │
             SPONTANEOUS BREATHING ─────────────────
                    TRIAL?
                        │
                        │ N
                        ▼
116 ──            (  END  )
```

FIG. 2

**FIG. 3**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006005433 A
- WO 2006131149 A